# EUROPEAN PATENT SPECIFICATION

(11) **EP 2 213 226 B1**
(45) Date of publication and mention of the grant of the patent: **13.06.2012**
(21) Application number: 09151930.6
(22) Date of filing: 03.02.2009
(51) Int. Cl.: A61B 5/024, A61B 5/08, A61B 5/00, A63B 23/18

(54) **System, method and device for auditory representation of rhythmical parameters**
System, Verfahren und Vorrichtung zur Hördarstellung rhythmischer Parameter
Système, procédé et dispositif pour la représentation auditive de paramètres rythmiques

(43) Date of publication of application: 04.08.2010
(73) Proprietor: Zimmermann, Heinrich, 3600 Thun (CH)
(72) Inventor: Zimmermann, Heinrich, 3600 Thun (CH)
(74) Representative: Scheuzger, Beat Otto

(56) References cited:
- EP-A1- 1 219 241
- US-A- 3 643 652
- US-A- 3 646 931
- US-A- 3 704 706
- US-A- 3 841 314
- US-A- 3 858 574
- US-A- 4 798 538
- US-A1- 2002 038 094
- US-A1- 2004 171 947
- US-A1- 2008 171 945
- US-A1- 2008 262 364
- US-B1- 6 379 309

## Description

The present invention relates to a system, method and device for creating an audible representation of rhythmical parameters such as the rhythmical physiological parameters of a living being.

In fields such as medical monitoring, intensive care, anaesthesia, fitness training, sport, relaxation training and bio-feedback, it is usual to monitor various physiological parameters which can give some indication of the physiological or psychological state of a person. Such rhythmical parameters include, for example, heart-beat, or the pulse measured at particular points in the body, or breathing (inhalation/exhalation). These examples are parameters which may be measured as physical quantities (acoustic signals, pressure, flow velocity, electrical potential, muscular activity, or position information about the position or movement of a body, etc), but other physiological parameters such as body temperature, brain activity or the chemical or hormonal constitution of blood or other fluids may also exhibit rhythmical characteristics. The characteristic of interest may be the frequency of the rhythmical parameter, or it may be the amplitude, or the pulse interval, or the occurrence of a particular signal pattern, or it may be any other recurring feature of the parameter.

For the sake of clarity and brevity, the invention is described in this application using the particular example of heart-rate and pulse monitoring of a human patient, but it should be understood that the invention applies equally to the monitoring of any rhythmical physiological parameter such as those examples given in the preceding paragraph. The invention is also applicable to the monitoring of rhythmical physiological parameters in living organisms other than human beings. The invention may also be used to aid communication of rhythmical parameters to blind or partially-sighted people. It may also be applied in the field of computer games, in which physiological parameters of a player may be used to influence the game, and musical education, in which physiological parameters of a person may be used to create or modify music.

The invention is particularly advantageous in applications where an audible representation with a frequency in a range suited to the human ear, for example from several hundred Hz to several kHz, is generated from a much lower-frequency signal, of the order of 0.1 Hz or 1 Hz or 10 Hz, for example. Furthermore, it has been found that audible representations with frequencies in the region of 400 Hz to 2 kHz are particularly advantageous, since the human ear is particularly sensitive to variations in frequency and/or other parameters of signals in this frequency range.

In the context of this application, the term "rhythmical parameter" is used to denote any recurring sequence of signals or events. The rhythmical features of the parameter may recur regularly or irregularly, and successive occurrences of the rhythmical features must be at least similar enough to establish that each occurrence belongs to the particular sequence.

It is well known (in the field of diagnostic medicine, for example) to represent physiological parameters as audible signals, so that a medical practitioner can receive information about the heart-rate of a patient, for example, without the need to view the information on a display. The advantage for the medical practitioner is that he or she can monitor the patient's heart-rate while continuing to perform other tasks. The human ear is particularly sensitive to even slight changes in a sound, and this sensitivity has been exploited in the use of audible tone generators representing such parameters.

The systems of the prior art invariably use some kind of sampling, integration or data-accumulation arrangement to establish the value which is to be audibly represented.

An example of a known system which audibly represents physiological parameters is described in United States patent application US4653498. The system described in this document includes input circuitry for sampling the pulsing oxygen saturation levels in the blood of a patient. A tone generator generates an intermittent audible tone whose pitch varies with the oxygen saturation level, with an intermittence rhythm determined by the pulse rate.

Such prior art systems suffer from the disadvantage that the information presented in audible form is merely an approximation of the parameter being measured (in the case of the above-cited document, the oxygenation pulse of a patient). Furthermore, there is an inherent time delay in the sampling, averaging and other processing which is performed before the audible tone is generated. Significant circuitry and/or microprocessor coding is also required to perform the averaging and other data processing functions of prior art systems. As is well known, increased circuit complexity means increased manufacturing cost, an increased risk of component or system failure, and a more bulky or weighty device (particularly disadvantageous in the case of a portable or wearable unit).

It is an object of the present invention to provide a system, method and device for audibly representing rhythmical parameters, in which the accuracy of the audible representation of the parameter is improved, and in which the delay in generating the audible representation of the parameter's value is reduced. It is also an object of the invention to provide such a system, method and device having reduced complexity.

US 3 858 574 and US 2002/038094 are considered relevant for the present invention. US 3 858 574 discloses the preamble of claims 1 and 7.

The above objects are met by the system of independent claim 7 and the method of independent claim 1. Additional variants of the invention are set out in the dependent claims.

The advantages of the present invention will become apparent in the detailed description of the embodiments, from which the inventive concept will be readily understood. The invention will be described with reference to the accompanying drawings, which are included for illustrative purposes only and should not be construed as limiting the scope of the invention.
Figure 1 shows a schematic representation of the function of a system according to a first embodiment of the invention.
Figure 2 shows an example of sensor placement according to a second embodiment of the invention.
Figure 3 shows in schematic form the function of a system according to the second embodiment of the invention.

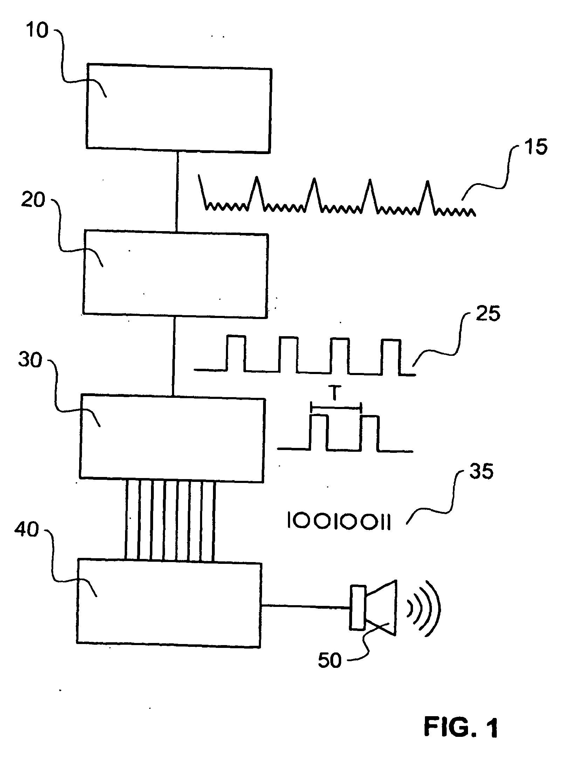

With reference to figure 1, a sensor (10) is shown, which may for example be a heart-beat sensor attached to a medical patient. A signal (15), such as the acoustic sound of a heartbeat, from a sensor (10), such as a microphone, is fed to a rhythmical pulse detector circuit (20), which identifies valid pulses in the waveform (15) and generates a standardized sequence of pulses (25) which is then used to characterize the pulse intervals (T) of successive pulses. Pulse interval encoder (30) generates a digital representation (35) of each pulse width (T) which is converted by an audio signal generator (40) into an audible signal which is fed to a loudspeaker or other suitable transducer (50).

In the case of the example illustrated in figure 1, the sensor output waveform (10) is depicted as being a regular periodic function, however a real waveform would normally contain variations of pulse width and/or shape. Rhythmical pulse detector circuit (20) serves to standardize the waveform to account for any such variations, such that the pulse intervals (T) between successive pulses in standardized pulse sequence (25) accurately represent the elapsed times between successive corresponding parts of waveform (15). A rhythmical pulse detector (20) is only required for the invention if the pulse (15) is not suitable for applying directly to the input of the pulse interval encoder (30), for example if the pulse (15) does not have a regular enough waveform, or does not comprise signal transitions or edges which would consistently trigger the input of the pulse interval encoder at the same point in each cycle of the waveform (15).

Each successive pulse width (T) is converted into a signal which accurately defines the pulse interval of the corresponding rhythmical parameter as it is detected. Figure 1 shows an 8-bit digital representation (35) of the encoded pulse interval (T), however in practice this encoding may be performed using any suitable encoding scheme. An analogue signal may be used instead of the digital signal shown, for example.

Audio signal generator (40) converts the encoded pulse interval signal (35) into an audio signal suitable for acoustic reproduction by a speaker or transducer (50). For example the pulse interval (T) may be used to determine the frequency (pitch) of the audio signal. The pitch may be inversely and/or linearly proportional to the pulse width, for example, or the function may be more complicated - for example a logarithmic or exponential function, or the function may be designed to emphasise greater variation in output frequency for pulse intervals of a particular range of values, thereby enhancing the sensitivity of the system to pulse intervals of those values. The function may be a mathematical calculation such as a polynomial function, or it may be a predetermined function defined by values stored in a mapping table. A simple multiplication function has been found to achieve effective results for pulse-rate monitoring, for example, with the reciprocal of each pulse interval (1/T) being multiplied by a suitable constant, such as 600 or 1000, to determine the audio frequency of the audible output. The audible signal which corresponds to each pulse interval (T) is then sounded by speaker (50) for the duration of the following pulse interval; upon the completion of this following pulse interval, the newly calculated audible signal then replaces the previous signal and is emitted by the speaker (50).

Varying the pitch is just one possible example of an audible representation of the rhythmical parameter, and it has the particular advantage that the human ear is particularly sensitive to changes in pitch. However, other audible effects can be used instead, such as the timbre or the amplitude (volume) of the audible signal. In cases where more than one parameter is being represented, such as, for example, pulse rate and blood oxygenation level, each parameter can be represented by a different characteristic of the audible signal. For example, one audible characteristic (eg frequency) being dependent on the pulse-rate, and a second (eg timbre) dependent on the oxygenation level.

The system described above and in figure 1 allows the almost instantaneous generation of an audio signal representing the pulse interval (T) of the rhythmical parameter being monitored. No sampling or averaging or other pulse-sequence-analysis needs to be performed, which means that the output audio signal is produced virtually as soon as the respective pulse interval it represents has finished. The output audio signal is also a faithful and accurate representation of the particular pulse interval which it represents, since no averaging or filtering is performed. In this way, the system of the invention generates a rapid and accurate signal representing the pulse interval (and thereby the instantaneous frequency) of the rhythmical parameter being monitored.

This virtual elimination of the delay can play a highly significant rôle in time-critical situations where, for example, a patient's pulse-rate falls suddenly and rapidly. Conventional systems, in which pulse intervals are averaged or sampled or integrated over a number of pulses, would significantly delay the output of audible information about the change in pulse-rate, partly because of the processing steps involved, and partly because an averaged value will inevitably lag behind the true instantaneous value of the pulse rate. The magnitude of the time lag will depend on the rate of change of the pulse rate and the averaging function being employed, but it is quite possible for the delay to be several tens of seconds. This is precious time for a patient in danger of brain damage due to acute cerebral hypoxia, for example.

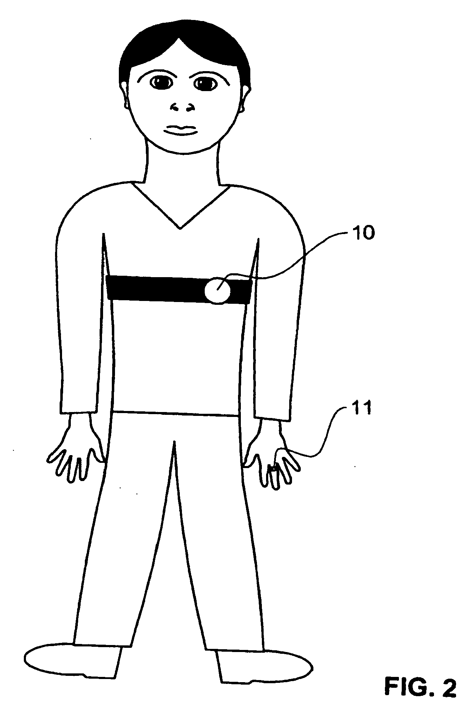

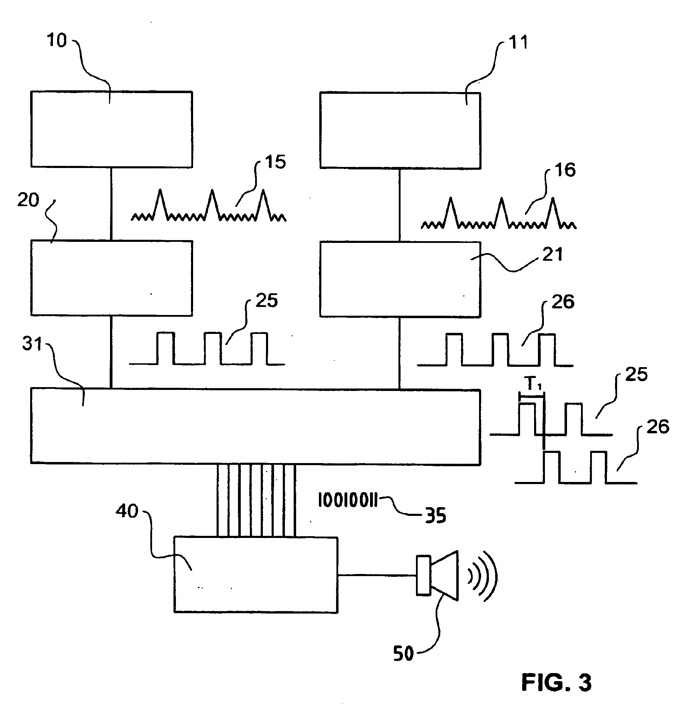

Figures 2 and 3 relate to a second embodiment of the invention, in which the parameter being audibly represented can be a propagation delay. This embodiment includes the elements of figure 1, but renumbered where necessary for the sake of clarity. In the example shown, two sensors (denoted 10 and 11) are used - one near a patient's heart and one on the patient's finger - to detect pulse signals. These detected pulse signals (15 and 16) are then shaped (if necessary) by rhythmical pulse detectors 20 and 21 and applied (for example as sequences of square pulses 25 and 26) to two respective inputs of a pulse propagation interval encoder (31), which generates a signal representing the delay between a pulse in the first pulse sequence (25) derived from the first sensor (10), and a corresponding pulse in the second pulse sequence (26) derived form the second sensor (11). This delay is denoted by T₁ in figure 3, and represents the time taken for a pulse to propagate from the first sensor (at the heart) to the second sensor (at the finger). This propagation delay is a useful indicator of the status of the patient's blood pressure. The interval T₁ is encoded by an audio signal generator (40) and reproduced audibly by a speaker or transducer (50). An eight-bit encoding is suggested in figure 3, but this could equally well be implemented using any other suitable form of coding, such as a sixteen-bit digital signal or an analogue signal.

Figure 2 shows two separate sensor devices (10) and (11) which can each be worn on the body, for example attached on a strap around the chest and/or to a finger, to detect pulse information of a person's body.

However, any number of sensors could be used, for example at various parts of the person's body, with the sensor outputs being inputted to a pulse interval generator capable of deriving rhythmical pulse interval information from the sensors according to a predetermined function which relates the outputs of the multiple sensor outputs. Alternatively, various combinations of sensors and/or sensor groups can be used, with each sensor or group being used to generate a different audible characteristic (frequency, timbre, volume etc) of the resulting audible signal.

The pulse detector circuitry, the pulse propagation interval encoder and/or the audio frequency generator can be integrated into one device worn on the body, optionally with one or more of the sensors integrated into the same device. Alternatively, the sensor(s) can be worn on the body and communicate with separate audio generating circuitry via wired or wireless communication means. In a preferred embodiment, the or each sensor is equipped with Bluetooth™ to communicate with a separate processing and audio generating device, for example implemented as an application running in a laptop or in a mobile phone. In this last example, the various signal processing and/or audio generating functions are performed using software algorithms instead of dedicated hardware, which has the advantage of being simpler and less costly to implement. Modern mobile phones are also equipped with relatively sophisticated audio-generation algorithms and speakers, which can be employed by the method of the invention to implement the required audio generation functions.

The audible signal generator may also be equipped with circuitry for rendering the audio output more pleasing to the ear. The transition from one pulse interval to the next, for example, can be smoothed slightly to avoid sudden changes in the audible signal.

The audible signal generator may also be provided with circuitry which, if at any time the pulse interval of the current pulse is already greater than the pulse interval of the preceding pulse, the audible signal generator already begins to modify the audio output (decrease the frequency, for example), before the end of the current pulse interval is detected. In this way, not only is there a smoother transition between the audible representation of one pulse interval and the next, but the listener is alerted earlier to the incidence of the longer pulse interval.

The audible signal generator may also be equipped with an alarm condition detector which can be triggered by, for example, a pulse interval which falls outside certain predetermined limits. This is useful, for example, to detect a condition where the patient's pulse rate falls below a certain predetermined threshold or stops altogether.

## Claims

1. Method of representing as variations in an audible tone, variations in rhythmical physiological parameter information from a living organism under analysis, the method comprising:
a first step of deriving from said living organism under analysis one or more input signals (15, 16) representing said rhythmical physiological parameter information and,
a second step of generating a sequence of standardized pulse signals (25, 26) from the or each input signal (15, 16),
The method being **characterized by**
a third step of, upon the generation of each standardized pulse signal in a first of said one or more sequences of standardized pulse signals (25, 26), determining a pulse interval (T, T₁) between the said each standardized pulse signal and a previously generated standardized pulse signal, and outputting a pulse interval signal (35) representing the length of the said each determined pulse interval (T, T₁), and
a fourth step of generating an audible signal having one or more audible characteristics determined as a predetermined function of the pulse interval signal (35), wherein the one or more audible characteristic are the audio frequency, the timbre and/or the volume of the audible signal.

2. Method according to claims 1, in which the said rhythmical parameter information is a physiological parameter of a human body, in particular an acoustic parameter, a pressure in a part of the body, the flow rate of a fluid in the body, an electrical potential in the body, a measure of the muscular activity in a body, or the position or movement of the body or a part of the body.

3. Method according to claim 2, in which the at least one input means (10, 11) include a first sensor means (10) for detecting a first input signal (15) representing rhythmical parameter information in a first part of the human body, and a second sensor means (11) for detecting a second input signal (16) representing rhythmical parameter information in a second part of the human body, and in which the pulse interval detection means (31) is adapted to measure the time interval (T₁) between a first pulse signal n a first one of said sequences of pulse signals (25), and a second pulse signal In a second one of said sequences of pulse signals (26).

4. Method according to any of the preceding claims, in which the said predetermined function is a linear function, a polynomial function or a function defined by a set of mapping data in a lookup table.

5. Method according to any of the preceding claims, in which at least one of the said input means (10, 11), pulse signal generating means (20. 21), pulse interval detection means (30, 31) and the audible signal generation means (40, 50) are integrated into a portable or wearable device.

6. Method according to any of the preceding claims, in which at least one of the pulse signal generating means (20, 21), the pulse interval detection means (30, 31) and the audible signal generation means (40, 50) are integrated into a first unit and in which the at least one sensor means (10, 11) and the first unit are equipped with means for communicating with each other via a wireless communications link.

7. System for representing variations in rhythmical physiological parameter information as variations in an audible tone, the system comprising:
at least one input means (10, 11) for inputting one or more input signals (15, 16) representing said physiological rhythmical parameter information and,
pulse signal generating means (20, 21) for generating a sequence of standardized pulse signals (25, 26) from the or each input signal (15, 16),
The system being **characterized by**
pulse interval detection means (30, 31) for receiving the or each said sequence of standardized pulse signals (25, 26) and, upon receipt of a received pulse signal in said the or each received sequence of pulse signals (25, 26), determining a pulse Interval (T, T₁) between the said received pulse signal and a previously received pulse signal, and outputting a pulse interval signal (35) representing the length of the said each determined pulse interval (T, T₁), and
audible signal generation means (40, 50) for generating, from the pulse interval signal (T, T₁) output by the pulse interval detection means (30, 31), an audible signal having one or more audible characteristics determined as a predetermined function of the pulse interval signal (35) wherein
the one or more audible characteristics are the audio frequency, the timbre and/or the volume of the audible signal.

8. System according to 7, in which the said rhythmical parameter information is a physiological parameter of a human body, in particular an acoustic parameter, a pressure in a part of the body, the flow rate of a fluid in the body, an electrical potential in the body, a measure of the muscular activity In a body, or the position or movement of the body or a part of the body.

9. System according to claim 8, in which the at least one Input means (10, 11) include a first sensor means (10) for detecting a first input signal (15) representing rhythmical parameter information in a first part of the human body, and a second sensor means (11) for detecting a second input signal (16) representing rhythmical parameter information in a second part of the human body, and in which the pulse interval detection means (31 is adapted to measure the time interval (T₁) between a first pulse signal in a first one of said sequences of pulse signals (25), and a second pulse signal in a second one of said sequences of pulse signals (26).

10. System according to one of claims 7 to 9, in which the said predetermined function is a linear function, a polynomial function, or a function defined by a set of mapping data in a lookup table.

11. System according to any of claims 7 to 10, in which at least one of the said input means (10, 11), pulse signal generating means (20, 21), pulse interval detection means (30, 31) and the audible signal generation means (40, 50) are integrated into a portable or wearable device.

12. System according to any of claims 7 to 11, in which at least one of the pulse signal generating means (20, 21), the pulse interval detection means (30, 31) and the audible signal generation means (40, 50) are integrated into a first unit and in which the at least one sensor means (10, 11) and the first unit are equipped with means for communicating with each other via a wireless communications link.

13. System according to claim 7, wherein the at least one input means (10, 11), the pulse signal generating means (20, 21), the pulse interval detection means (30, 31) and the audible signal generation means (40, 50) are integrated into one device.

## Patentansprüche

1. Verfahren zum Darstellen von Änderungen rhythmischer physiologischer Parameter-Informationen von unter Analyse stehenden lebenden Organismen als Änderungen eines hörbaren Tons, mit:
einem ersten Schritt, um von dem lebenden Organismus unter Analyse ein oder mehrere Eingabesignale (15, 16) herzuleiten, welche die rhythmische physiologische Parameter-Informationen darstellen; und
einem zweiten Schritt, um von dem oder jedem Eingabesignal (15, 16) eine Sequenz standardisierter Impulssignale (25, 26) zu erzeugen,
**gekennzeichnet durch**
einen dritten Schritt, um nach dem Erzeugen jedes standardisierten Impuls-Signals in einer ersten Sequenz der einen oder mehreren Sequenzen standardisierter Impulssignale (25, 26) ein Impulsintervall (T, T₁) zu bestimmen zwischen diesem jedem standardisierten Impulssignal und einem zuvor erzeugten standardisierten Impulssignal, und um ein Impulsintervall-Signal (35) auszugeben, welches die Länge dieses jeden bestimmten Impulsintervalls (T, T1) darstellt; und
einen vierten Schritt zum Erzeugen eines hörbaren Signals mit einer oder mehreren, als vorbestimmte Funktion des Impulsintervall-Signals (35) bestimmten, hörbaren Kenngrössen, wobei die eine oder die mehreren hörbaren Kenngrössen die Tonfrequenz, die Klangfarbe und/oder die Lautstärke des hörbaren Signals sind.

2. Verfahren nach Anspruch 1, bei welchem die rhythmische physiologische Parameter-Information ein physiologischer Parameter eines menschlichen Körpers ist, insbesondere ein akustischer Parameter, ein Druck in einem Teil des Körpers, die Strömungsgeschwindigkeit eines Fluids in dem Körper, ein elektrisches Potential in dem Körper, ein Mass der Muskelaktivität in einem Körper, oder die Lage oder Bewegung des Körpers oder eines Teils des Körpers.

3. Verfahren nach Anspruch 2, bei welchem das mindestens eine Eingabemittel (10, 11) ein erstes Sensormittel (10) zum Erfassen eines ersten Eingabesignals (15) enthält, welches rhythmische Parameter-Informationen in einem ersten Teil des menschlichen Körpers darstellt, und ein zweites Sensormittel (11) zum Erfassen eines zweiten Eingabesignals (16) enthält, welches rhythmische Parameter-Informationen in einem zweiten Teil des menschlichen Körpers darstellt; und bei welchem das Impulsintervall-Erfassungsmittel (31) dazu ausgelegt ist, um das Zeitintervall (T₁) zu messen zwischen einem ersten Impulssignal in einer ersten Sequenz der Sequenzen von Impulssignalen (25) und einem zweiten Impulssignal in einer zweiten Sequenz der Sequenzen von Impulssignalen (26).

4. Verfahren nach einem der vorhergehenden Ansprüche, bei welchem die vorbestimmte Funktion eine lineare Funktion, eine Polynom-Funktion oder eine durch einen Satz von Abbildungsdaten in einer Nachschlagetabelle definierte Funktion ist.

5. Verfahren nach einem der vorhergehenden Ansprüche, bei welchem mindestens eines der Eingabemittel (10, 11), Impulssignal-Erzeugungsmittel (20, 21), Impulsintervall-Erfassungsmittel (30, 31) und Hörsignal-Erzeugungsmittel (40, 50) in eine insbesondere am Körper tragbare Vorrichtung integriert ist bzw. sind.

6. Verfahren nach einem der vorhergehenden Ansprüche, bei welchem mindestens eines der Impulssignal-Erzeugungsmittel (20, 21), Impulsintervall-Erfassungsmittel (30, 31) und Hörsignal-Erzeugungsmittel (40, 50) in eine erste Einheit integriert ist bzw. sind; und bei welchem das mindestens eine Sensormittel (10, 11) und die erste Einheit mit einem Mittel ausgestattet sind, um miteinander über eine drahtlose Kommunikations-Verbindung zu kommunizieren.

7. System zum Darstellen von Änderungen rhythmischer physiologischer Parameter-Informationen als Änderungen eines hörbaren Tons, mit:
mindestens einem Eingabemittel (10, 11) zum Eingeben eines oder mehrerer Eingabesignale (15, 16), welche die rhythmische physiologische Parameter-Informationen darstellen; und
einem Impulssignal-Erzeugungsmittel (20, 21) zum Erzeugen einer Sequenz standardisierter Impulssignale (25, 26) von dem oder jedem Eingabesignal (15, 16),
**gekennzeichnet durch**
ein Impulsintervall-Erfassungsmittel (30, 31) zum Empfangen der Sequenz oder jeder Sequenz standardisierter Impulssignale (25, 26), und um nach dem Empfangen eines empfangenen Impulssignals in der oder jeder empfangenen Sequenz von Impulssignalen (25, 26) ein Impulsintervall (T, T₁) zu bestimmen zwischen dem empfangenen Impulssignal und einem zuvor empfangenen Impulssignal, und zum Ausgeben eines Impulsintervall-Signals (35), welches die Länge dieses jeden bestimmten Impulsintervalls (T, T1) darstellt; und
einem Hörsignal-Erzeugungsmittel (40, 50), um von dem **durch** das Impulsintervall-Erfassungsmittel (30, 31) ausgegebenen Impulsintervall-Signal (T, T₁) ein hörbares Signal mit einer oder mehreren, als vorbestimmte Funktion des Impulsintervall-Signals (35) bestimmten, hörbaren Kenngrössen, wobei die eine oder mehreren hörbaren Kenngrössen die Tonfrequenz, die Klangfarbe und/oder die Lautstärke des hörbaren Signals sind.

8. System nach Anspruch 7, bei welchem die rhythmische Parameter-Information ein physiologischer Parameter eines menschlichen Körpers ist, insbesondere ein akustischer Parameter, ein Druck in einem Teil des Körpers, die Strömungs-geschwindigkeit eines Fluids in dem Körper, ein elektrisches Potential in dem Körper, ein Mass der Muskelaktivität in einem Körper, oder die Lage oder Bewegung des Körpers oder eines Teils des Körpers.

9. System nach Anspruch 8, bei welchem das mindestens eine Eingabemittel (10, 11) ein erstes Sensormittel (10) zum Erfassen eines ersten Eingabesignals (15) enthält, welches rhythmische Parameter-Informationen in einem ersten Teil des menschlichen Körpers darstellt, und ein zweites Sensormittel (11) zum Erfassen eines zweiten Eingabesignals (16) enthält, welches rhythmische Parameter-Informationen in einem zweiten Teil des menschlichen Körpers darstellt; und bei welchem das Impulsintervall-Erfassungsmittel (31) dazu ausgelegt ist, um das Zeitintervall (T₁) zu messen zwischen einem ersten Impulssignal in einer ersten Sequenz der Sequenzen von Impulssignalen (25) und einem zweiten Impulssignal in einer zweiten Sequenz der Sequenzen von Impulssignalen (26).

10. System nach einem der Ansprüche 7 bis 9, bei welchem die vorbestimmte Funktion eine lineare Funktion, eine Polynom-Funktion oder eine durch einen Satz von Abbildungsdaten in einer Nachschlagetabelle definierte Funktion ist.

11. System nach einem der Ansprüche 7 bis 10, bei welchem mindestens eines der Eingabemittel (10, 11), Impulssignal-Erzeugungsmittel (20, 21), Impulsintervall-Erfassungsmittel (30, 31) und Hörsignal-Erzeugungsmittel (40, 50) in eine, insbesondere am Körper, tragbare Vorrichtung integriert ist bzw. sind.

12. System nach einem der Ansprüche 7 bis 11, bei welchem mindestens eines der Impulssignal-Erzeugungsmittel (20, 21), Impulsintervall-Erfassungsmittel (30, 31) und Hörsignal-Erzeugungsmittel (40, 50) in eine erste Einheit integriert ist bzw. sind; und bei welchem das mindestens eine Sensormittel (10, 11) und die erste Einheit mit einem Mittel ausgestattet sind, um miteinander über eine drahtlose Kommunikations-Verbindung zu kommunizieren.

13. System nach Anspruch 7, bei welchem das mindestens eine Eingabemittel (10, 11), das Impulssignal-Erzeugungsmittel (20, 21), das Impulsintervall-Erfassungsmittel (30, 31) und das Hörsignal-Erzeugungsmittel (40, 50) in eine einzige Vorrichtung integriert ist bzw. sind.

## Revendications

1. Méthode pour représenter, comme variations d'un ton audible, des variations d'information-paramètre physiologique rythmique d'un organisme vivant sous analyse, la méthode comprenant:
une première étape pour dériver dudit organisme vivant sous analyse un ou plusieurs signaux d'entrée (15, 16) représentant ladite information-paramètre physiologique rythmique, et
une seconde étape pour générer une séquence de signaux standardisés d'impulsion (25, 26) à partir du ou de chaque signal d'entrée (15, 16),
**caractérisée par**
une troisième étape pour, après la génération de chaque signal standardisé d'impulsion dans une première séquence desdites une ou plusieurs séquences de signaux standardisés d'impulsion (25, 26), déterminer un intervalle d'impulsion (T, T₁) entre ledit chaque signal standardisé d'impulsion et un signal standardisé d'impulsion généré préalablement, et produire en sortie un signal d'intervalle-impulsion (35) représentant la longueur de chaque intervalle-impulsion déterminé (T, T₁), et
une quatrième étape pour générer un signal audible avec une ou plusieurs caractéristiques audibles déterminées comme fonction prédéterminée dudit signal d'intervalle-impulsion (35), ladite une ou plusieurs caractéristiques audibles étant données par l'audio-fréquence, le timbre et/ou le volume dudit signal audible.

2. Méthode selon la revendication 1, dans laquelle ladite information-paramètre physiologique rythmique est un paramètre physiologique d'un corps humain, notamment un paramètre acoustique, une pression dans une partie du corps, la vitesse d'écoulement d'un fluide dans le corps, un potentiel électrique dans le corps, une mesure de l'activité musculaire dans un corps, ou la position ou le mouvement du corps ou d'une partie du corps.

3. Méthode selon la revendication 2, dans laquelle ledit au moins un moyen d'entrée (10, 11) comporte un premier moyen capteur (10) pour détecter un premier signal d'entrée (15) représentant de l'information-paramètre rythmique dans une première partie du corps humain, et un second moyen capteur (11) pour détecter un second signal d'entrée (16) représentant de l'information-paramètre rythmique dans une seconde partie du corps humain; et dans laquelle ledit moyen détecteur d'intervalle d'impulsion (31) est adapté pour mesurer l'intervalle de temps (T₁) entre un premier signal d'impulsion dans une première séquence desdites séquences de signaux d'impulsion (25) un second signal d'impulsion dans une seconde séquence desdites séquences de signaux d'impulsion (26).

4. Méthode selon l'une quelconque des revendications précédentes, dans laquelle ladite fonction prédéterminée est une fonction linéaire, une fonction de polynôme ou une fonction définie par un ensemble de données de mappage dans une table de correspondance (LUT).

5. Méthode selon l'une quelconque des revendications précédentes, dans laquelle au moins un desdits moyens d'entrée (10, 11), moyens générateurs de signal d'impulsion (20, 21), moyens détecteurs d'intervalle d'impulsion (30, 31) et moyens générateurs de signal audible (40, 50) sont intégrés dans un dispositif portable, notamment portable au corps.

6. Méthode selon l'une quelconque des revendications précédentes, dans laquelle au moins un desdits moyens générateurs de signal d'impulsion (20, 21), moyens détecteurs d'intervalle d'impulsion (30, 31) et moyens générateurs de signal audible (40, 50) sont intégrés dans une première unité; et dans laquelle ledit au moins un moyen capteur (10, 11) et ladite première unité sont dotés d'un moyen pour communiquer l'un avec l'autre via une connexion de communication sans fil.

7. Système pour représenter des variations d'information-paramètre physiologique rythmique comme variations d'un ton audible, le système comprenant:
au moins un moyen d'entrée (10, 11) pour entrer un ou plusieurs signaux d'entrée (15, 16) représentant ladite information-paramètre physiologique rythmique; et
un moyen générateur de signal d'impulsion (20, 21) pour générer une séquence de signaux standardisés d'impulsion (25, 26) dudit ou de chacun desdits signaux d'entrée (15, 16),
**caractérisé par**
un moyen détecteur d'intervalle d'impulsion (30, 31) pour recevoir ladite séquence ou chacune desdites séquences de signaux standardisés d'impulsion (25, 26), et, après la réception d'un signal d'impulsion reçu dans ladite ou chacune desdites séquences reçues de signaux standardisés d'impulsion (25, 26), déterminer un intervalle d'impulsion (T, T₁) entre ledit signal reçu d'impulsion et un signal d'impulsion reçu préalablement, et produire en sortie un signal d'intervalle-impulsion (35) représentant la longueur de chaque intervalle-impulsion déterminé (T, T₁); et
un moyen générateur de signal audible (40, 50) pour générer, à partir dudit signal d'intervalle-impulsion (T, T₁) produit en sortie par ledit moyen détecteur d'intervalle-impulsion, un signal audible avec une ou plusieurs caractéristiques audibles déterminées comme fonction prédéterminée dudit signal d'intervalle-impulsion (35), ladite une ou plusieurs caractéristiques audibles étant données par l'audio-fréquence, le timbre et/ou le volume dudit signal audible.

8. Système selon la revendication 7, dans lequel ladite information-paramètre rythmique est un paramètre physiologique d'un corps humain, notamment un paramètre acoustique, une pression dans une partie du corps, la vitesse d'écoulement d'un fluide dans le corps, un potentiel électrique dans le corps, une mesure de l'activité musculaire dans un corps, ou la position ou le mouvement du corps ou d'une partie du corps.

9. Système selon la revendication 8, dans lequel ledit au moins un moyen d'entrée (10, 11) comporte un premier moyen capteur (10) pour détecter un premier signal d'entrée (15) représentant de l'information-paramètre rythmique dans une première partie du corps humain, et un second moyen capteur (11) pour détecter un second signal d'entrée (16) représentant de l'information-paramètre rythmique dans une seconde partie du corps humain; et dans lequel ledit moyen détecteur d'intervalle d'impulsion (31) est adapté pour mesurer l'intervalle de temps (T₁) entre un premier signal d'impulsion dans une première séquence desdites séquences de signaux d'impulsion (25) un second signal d'impulsion dans une seconde séquence desdites séquences de signaux d'impulsion (26).

10. Système selon l'une quelconque des revendications 7 à 9, dans lequel ladite fonction prédéterminée est une fonction linéaire, une fonction de polynôme ou une fonction définie par un ensemble de données de mappage dans une table de correspondance (LUT).

11. Système selon l'une quelconque des revendications 7 à 10, dans lequel au moins un desdits moyens d'entrée (10, 11), moyens générateurs de signal d'impulsion (20, 21), moyens détecteurs d'intervalle d'impulsion (30, 31) et moyens générateurs de signal audible (40, 50) sont intégrés dans un dispositif portable, notamment portable au corps.

12. Système selon l'une quelconque des revendications 7 à 11, dans lequel au moins un desdits moyens générateurs de signal d'impulsion (20, 21), moyens détecteurs d'intervalle d'impulsion (30, 31) et moyens générateurs de signal audible (40, 50) sont intégrés dans une première unité; et dans lequel ledit au moins un moyen capteur (10, 11) et ladite première unité sont dotés d'un moyen pour communiquer l'un avec l'autre via une connexion de communication sans fil.

13. Système selon la revendication 7, dans lequel ledit au moins un moyen d'entrée (10, 11), ledit moyen générateur de signal d'impulsion (20, 21), ledit moyen détecteur d'intervalle d'impulsion (30, 31) et ledit moyen générateur de signal audible (40, 50) sont intégrés dans un seul dispositif.
